Europäisches Patentamt

(19) European Patent Office

Office européen des brevets

(11) Publication number: **0 152 292**

**A2**

(12) # EUROPEAN PATENT APPLICATION

(21) Application number: 85300870.4

(22) Date of filing: 08.02.85

(51) Int. Cl.⁴: **A 61 K 9/48**
A 61 K 31/165, A 61 K 47/00

(30) Priority: 08.02.84 US 578070

(43) Date of publication of application:
21.08.85 Bulletin 85/34

(84) Designated Contracting States:
AT BE CH DE FR GB IT LI NL SE

(71) Applicant: R. P. SCHERER CORPORATION
2075 W. Big Beaver Road
Troy, MI 48099(US)

(72) Inventor: Rogers, Garnell A., Jr.
1925 Chesapeake Court
Oldsmar Florida 33557(US)

(74) Representative: Lamb, John Baxter et al,
Marks & Clerk 57/60 Lincoln's Inn Fields
London WC2A 3LS(GB)

(54) Acetaminophen gelatin capsules.

(57) A gelatin capsule for oral administration comprises a gelatin shell substantially encapsulating a physiologically compatible fill material, the fill material comprising an effective dosage amount of acetaminophen, a vehicle for the acetaminophen, a surfactant and a modifier for the vehicle, characterised in that:-

(a) the vehicle comprises a low molecular weight polyethylene glycol having an average molecular weight of from about 400 to 1000 and a higher molecular weight polyethylene glycol having an average molecular weight of from about 1000 to about 10000, the acetaminophen being substantially evenly distributed through the vehicle;

(b) the surfactant is of a type that is physiologically soluble and is in solution in the vehicle; and

(c) the viscosity modifier is present in an amount equal to or greater than the amount necessary to gel the vehicle.

EP 0 152 292 A2

M&C FOLIO: 799P49593                    WANGDOC: 0221i

## Acetaminophen gelatin capsules

The present invention relates to gelatin capsule formulations for the oral delivery of acetaminophen to warm-blooded mammals.

Acetaminophen (N-acetyl-P-aminophenol or paracetamol) is a drug widely used as a nonprescription analgesic. Like aspirin, acetaminophen acts as an antipyretic and an analgesic, but does not cause some of the undesirable side effects of aspirin.

..An important consideration in the therapeutic application of acetaminophen is the time of onset of therapeutic action following oral administration, such as by swallowing a tablet or capsule, usually with the aid of water. It is highly desirable from the patient's point of view that the drug begin to relieve pain or reduce fever as quickly as possible. Therefore, the development of a capsule containing an effective unit dosage amount of acetaminophen that provides more rapid therapeutic onset would be significant.

Many acetaminophen products marketed today are in the form of power filled, hard shell gelatin capsule products. While these known capsules adequately deliver acetaminophen to human physiological systems, the time necessary for disintegration and dissolution of the powder dosage form of the acetaminophen can delay the absorption of the acetaminophen from the gastrointestinal fluids and consequently delay the onset of therapeutic action. [Hedges et al., "A Comparison of the Absorption of Effervescent Preparations of Paracetamol and Penicillin V (Phenoxymethylpenicillin) with Solid Dise Forms of These Drugs," _Journal of Clincal Pharmacology_ (July, 1974): 363-368].

Soft elastic gelatin capsules are widely used as an alternative to powder filled, hard shell capsules for the oral administration of pharmaceuticals, vitamin, dietary supplements and the like. It is also possible to use conventional hard shell gelatin capsules filled with viscous oil or paste formulations as an alternative to powder filled, hard shell capsules. [Elanco Products Co., "Technology of Filling Oil or Paste Formulations Into Hard Gelatin Capsules," October, 1982.] However, soft elastic gelatin capsules are often preferred by patients since they are easier to swallow than conventional hard gelatin capsules. [Delaney, "Surveying Consumer Preferences," _Pharmaceutical Executive_, _2_: 34, 36, 38 (1982)]. In the case of acetaminophen, one study has shown that a particular soft gelatin capsule containing an acetaminophen

3                    0152292

formulation had an inferior rate of absorption when
compared to conventional hard tablets.  [Albert et al.,
"Bioavailability Studies of Acetaminophen and
Nitrofurantoin," Journal of Clinical Pharmacology
(May-June, 1974): 264-270.]  If a soft elastic gelatin
capsule containing acetaminophen were developed that had
a superior absorption rate, the resulting capsule would
combine rapid onset and easy oral administration.
Therefore, there is a need for a readily swallowable
soft elastic gelatin capsule containing an effective
unit dosage amount of acetaminophen characterised by
more rapid onset of thereapeutic action than
conventional powder filled, hard gelatin capsules.

It is an object of the present invention to provide
a gelatin capsule containing an effective unit dosage
amount of acetaminophen that provides rapid onset of
therapeutic activity upon oral administration of the
capsule.

A unique fill material for a hard shell or soft
elastic gelatin capsule has now been discovered that
contains an effective unit dosage amount of
acetaminophen.  Unexpectedly, this unique fill material
exhibits a reduced time required to reach maximum plasma
acetaminophen concentration ($T_{max}$) upon oral ingestion
as compared with powder filled, hard shell gelatin

capsules. Since $T_{max}$ is directly related to the rapidity of therapeutic onset, the inventive formulations provide a gelatin capsule containing an effective unit dosage amount of acetaminophen that delivers the analgesic and antipyretic effects of the acetaminophen more rapidly than conventional powder filled hard shell capsules.

It is a specific object of the present invention to provide a gelatin capsule comprising a gelatin shell encapsulating a fill material including an effective unit dosage amount of acetaminophen, a vehicle for the acetaminophen, a surfactant, and a viscosity modifier. The resulting fill material is physiologically compatible.

According to the invention there is provided a gelatin capsule for oral administration comprising a gelatine shell substantially encapsulating a physiologically compatible fill material, the fill material comprising an effective dosage amount of acetaminophen, a vehicle for the acetaminophen, a surfactant and a modifier for the vehicle, characterised in that:-

(a) the vehicle comprises a low molecular weight polyethylene glycol having an average molecular weight of from about 400 to 1000 and a higher molecular weight polyethylene glycol having an average molecular weight of from about 1000 to about 10000, the acetaminophen being substantially evenly distributed through the vehicle;

(b) the surfactant is of a type that is physiologically soluble and is in solution in the vehicle; and

(c) the viscosity modifier is present in an amount equal to or greater than the amount necessary to gel the vehicle.

The vehicle for the acetaminophen includes a low molecular weight and a higher molecular weight polyethylene glycol. The low molecular weight polyethylene glycol desirably has an average molecular weight of about 400 to about 1000, while the higher molecular weight polyethylene glycol has an average molecular weight of about 1000 to 8000. For use with soft elastic gelatin shells, the weight ratio of the low molecular weight polytheylene glycol to the higher molecular weioght polyethylene glycol should advantageously be from about 1:1 to about 40:1. For use with hard gelatin shells, the weight ratio of the low

6                    0152292

molecular weight polyethylene glycol to the higher
molecular weight polyethylene glycol should
advantageously be from about 0:1 to about 20:1.  The
effective unit dosage amount of acetaminophen is then
substantially evenly distributed throughout the vehicle.

The incorporation of a surfactant aids the even
distribution of acetaminophen within the vehicle and,
more importantly, speeds the dissolution of the
acetaminophen in the gastrointestinal fluids upon
ingestion.  The surfactant is dissolved within the
vehicle and must be soluble in the gastric fluids.  A
viscosity modifier is also present that is capable of
gelling the polyethylene glycol vehicle.  The viscosity
modifier must be present in an amount equal to or
greater than the amount necessary to substantially gel
the vehicle.  The resulting formulation provides a
gelatin capsule containing an effective unit dosage
amount of acetaminophen that produces rapid therapeutic
blood levels upon oral ingestion.

Various means of manufacturing soft elastic gelatin
capsules are well known in the pharmaceutical art.  The
novel fill material of the present invention may be used
with any conventional soft elastic gelatin shell that is
capable of substantially encapsulating the fill material
for a reasonable period of time.

In a preferred aspect of the present invention, the fill material is filled into a soft elastic gelatin shell that encloses the fill material. The fill material does not attack the walls of the one piece, soft elastic gelatin capsule. The gelatin capsule shell is formulated in accordance with conventional techniques for making filled, soft elastic gelatin capsules containing therapeutically effective unit dosage amounts of an active drug ingredient. In one conventional shell formulation, there is included about 30-53 parts by weight of gelatin, about 15-48 parts by weight of a plasticizer, such as glycerin or sorbitol, and about 16-40 parts by weight of water. Additionally, the gelatin shell may contain preservatives, such as mixed parabens, ordinarily methyl or propyl parabens, in about 4:1 ratio. The parabens may be incorporated in the shell formulation in minor proportions as compared to the total weight of the shell formulation. Conventional gelatin capsules utilize gelatin having a Bloom value of about 160-200 although this value may be varied over a wider range. In a conventional manner, the gelatin composition is mixed and melted under vacuum conditions. The capsules may be simultaneously formed and filled using conventional methods and apparatus such as disclosed, for example, in U.S. Patent Nos. 1,970,396; 2,288,327; and 2,318,718. The gelatin capsules are formed into the desired shape and size so that they can be readily swallowed, usually with the aid of water. The resulting capsule is soluble in water and in gastrointestinal juices. Upon swallowing, the gelatin shell rapidly dissolves in the stomach thereby introducing the fill material into the physiological system.

In another aspect of the present invention, the fill material is filled into a hard shell gelatin capsule of the type generally comprising two halves that are telescopically interconnected together in various ways, such as by the use of friction. One type of locking hard shell gelatin capsule is shown, for example, in U.S. Patent No. 4,040,536.

The techniques and apparatus that are employed to manufacture such filled, hard shell gelatin capsules generally, are quite well known and established. The manufacture of hard shell gelatin capsules is most generally performed by equipment known in the art as a "Colton 950" machine. In manufacturing the gelatin capsule shells, rigid pins are supported on a rigid support member, and are dipped into a liquid gelatin which coats each of the pins. The gelatin is dried to a desired moisture content while being conveyed through a drying chamber. The formed gelatin shell half is stripped from each of the pins and cut to the desired size. The shell halves, that is, the body half and the cap half, are joined together, prior to filling, and packaged. The empty joined shells are then filled by a manufacturer. Prior to filling, the empty shell halves must be separated. The body portion is then filled with the medicament, vitamins, or the like and the cap portion of the shell is thereafter rejoined to the filled body portion. The capsules, once joined, are held together generally by friction or locking indentations along the overlapping portions of the cap and body portions of the shell so to provide the desired securement. The present viscous fill material may be filled into manufactured hard gelatin shells in any of the ways well known in the art, such as by using a Rotop 8 filler machine marketed by Elanco Products Co., Indianapolis, Indiana 46285. [See, Elanco Products Co., "Technology of Filling Oil or Paste Formulations into Hard Gelatin Capsules," October, 1982.]

The acetaminophen used in the fill material is preferably in powder form and most preferably has a particle size of U.S. standard 80 mesh or less. Coarse acetaminophen particles are as generally compatible with the conventional machinery used to produce gelatin capsules since they tend to adversely affect the pumpability of the fill material. Furthermore, large particle size reduces the dissolution time for the acetaminophen in the gastrointestinal fluids. Longer dissolution time results in a longer onset time for the acetaminophen's therapeutic effects. Though any effective unit dosage amount of acetaminophen may be used in the context of the present invention, a common dosage range is 325-650 milligrams. A dosage of 500 milligrams is generally most desirable.

A preferred vehicle for the acetaminophen powder includes two polyethylene glycols having different average molecular weights. The first polyethylene glycol has a relatively low molecular weight range of about 400 to about 1000, preferably, about 400 to about 600, and most preferably, about 400. The second polyethylene glycol has a higher molecular weight range of about 1000 to about 8000, preferably from about 1450 to about 8000, and most preferably, about 3350.

The use of two polyethylene glycols is significant, since a single higher molecular weight polyethylene glycol generally cannot balance the need for pumpability in the manufacturing process with the need for stability of the produced capsule. The unique combination of two polyethylene glycols of different molecular weights in preselected proportions yields a vehicle that melts at the proper temperature, retains pumpability and results in a stable product.

11

For use in soft elastic gelatin shells, the weight ratio of the low molecular weight polyethylene glycol to the higher molecular weight polyethylene glycol is desirably about 40:1 to about 1:1, preferably from 25:1 to about 15:1 and most preferably about 20:1. If the ratio is significantly above 40:1, the vehicle does not melt at the proper temperature. If the ratio is significantly below 1:1, the vehicle generally becomes too viscous and therefore unpumpable at the temperatures conventionally used in the manufacture of soft elastic gelatin capsules.

For use in hard gelatin shells, the weight ratio of low molecular weight polyethylene to the higher molecular weight polyethylene is suitably about 20:1 to about 0.1:, preferably from about 15:1 to about 0:1 and most preferably about 10:1. If the ratio goes significantly above 20:1, the vehicle would tend to leak out of the hard shell capsule.

The low molecular weight polyethylene glycol should not have an average molecular weight much below 400 since such polyethylene glycols tend to diffuse through the shells of conventional gelatin capsules. In turn, the use of polyethylene glycols with average molecular weights much above 1000 tends to result in an unpumpable vehicle. On the other hand, use of polyethylene glycols having molecular weights above about 8000 makes it difficult to blend the higher molecular weight polyethylene glycol with the low molecular weight polyethylene glycol.

To assure rapid onset of therapeutic effect, the acetaminophen is thoroughly distributed within the vehicle. It is most preferably that at least a portion of the acetaminophen be dissolved within the vehicle so that the vehicle is supersaturated with acetaminophen. The excess acetaminophen then remains in suspension within the vehicle. However, the present invention is also directed to fill materials where only minor amounts of acetaminophen are dissolved in the vehicle so that generally all of the acetaminophen remains suspended.

The surfactant surrounds and separates the acetaminophen particles suspended in the vehicle and also aids the rapid dissolution of the acetaminophen in the gastrointestinal fluids upon oral adminstration of the capsule. The surfactant is soluble in gastrointestinal fluids (i.e. it is physiologically soluble) and is also dissolved within the vehicle. Preferably, the surfactant is present in an amount from about 0.01 to about 1 percent based on the total weight of the fill material. Most preferably, the surfactant should be present in an amount of about 0.5 percent by total weight of the fill material. A preferred

13    **0152292**

surfactant is polysorbate 80 NF.  However, other appropriate surfactants may be used successfully with the present invention as long as they are physiologically compatible, soluble in gastric fluids, and soluble in the vehicle.

The amount of surfactant is desirably optimized to achieve a balance between increased therapeutic onset and increased pumpability in the particular manufacturing process.  If an insufficient amount of surfactant is used, the optimum rate of therapeutic onset is not achieved.  On the other hand, if too much surfactant is present, it can adversely affect the pumpability of the fill material.  Also, it may inhibit the processes used to seal the two halves of a soft elastic gelatin shell to form a soft elastic gelatin capsule.

The viscosity modifier is present in the soft elastic gelatin capsule formulation primarily to increase the temperature stability and shelf life of the fill material.  In the hard shell gelatin capsule application, the viscosity modifier also acts to prevent or retard capsule leakage.  The viscosity modifier used is capable of gelling the polyethylene glycol vehicle and is present in an amount sufficient to gel substantially all of the vehicle.  One preferred viscosity modifier is Carbopol 934-P, a tradename of the B.F. Goodrich Co., Cleveland, Ohio, 44131 for an acrylic acid resin having an average molecular weight of about 3,000,000 and having a high carboxylic content. Another preferred viscosity modifier is fumed silica that serves both as a gelling agent and as a gelling agent and as a glidant for the fill material during the manufacturing process. Though these two viscosity modifiers are preferred, other viscosity modifiers may be used that are capable of gelling the vehicle.

When Carbopol 934-P is used, it should preferably be present in an amount from 0 to 1 percent based on the total weight of the fill material. Similarly, fumed silica should be present in an amount from 0 to about 3 percent based on the total fill material weight. Most preferably, a combination of Carbopol 934-P and fumed silica should be used containing about 0.1 percent Carbopol 934-P and about 0.3 percent fumed silica based on the total weight of the fill material. Such a combination delays the onset of gelatin for at lest 24 hours allowing the fill material to be easily heated and pumped during the manufacturing process. Once the vehicle has completely gelled, the resulting fill material has enhanced temperature stability for more consistent and longer shelf life.

0152292

Though not required, propylene glycol or glycerin or a mixture thereof is desirably added to the soft elastic gelatin capsule fill material to reduce any undesirable interactions between the polyethylene glycol vehicle and the soft elastic gelatin shell.  Propylene glycol and glycerin act as humectants by setting up as an equilibrium between the moisture content of the vehicle and the soft elastic gelatin capsule shell.  This prevents the polyethylene glycol vehicle from removing excessive amounts of water from the soft elastic gelatin shell.  In like fashion, propylene glycol and glycerin act as equilibrium plasticizers so that the polyethylene glycerin act as equilibrium plasticizers so that the polyethylene glycol vehicle does not remove excessive amounts of plastizers from the soft elastic gelatin shell.  Thus, propylene glycol and glycerin prevent the polyethylene glycol vehicle from rendering the soft elastic gelatin capsule hard, brittle, and subject to damage during handling.  Preferably propylene glycol or glycerin is present in an amount from 0 to about 10 percent based on the total weight of the fill material. Most preferably, propylene glycol is present in an amount of about 0.5 percent by total fill material weight.  Since equilibrium plasticizers and humectants are not necessary or desirable for use with hard shell gelatin capsule formulations, neither propylene glycol nor glycerin should advantageously be used with the inventive fill material for hard shell gelatin capsules, though their inclusion is not prohibited.

A significant feature of the novel fill material of the present invention for use with soft elastic gelatin shells is that it is nondilatant. Nondilatant materials become more fluid or less viscous when they are agitated by stirring or shaking. By contrast, dilatant materials increase in viscosity with agitation. Nondilatant fill materials are compatible with conventional manufacturing processes for soft elastic gelatin capsules. Dilatant materials tend to clog the machinery carrying the fill material. Nondilatant materials are easier to pump since they become less viscous when force is exerted upon them. Though many different combinations of the components of the inventive fill materials may be formulated within the scope fo the present invention for use in soft elastic gelatin capsules, the resulting fill materials are nondilatant in character. Fill materials for use in hard shell filling may be either dilatant or nondilatant in character.

Example 1

A fill material having the following composition per capsule was prepared.

| Ingredients | Milligrams |
|---|---|
| Polyethylene glycol 400 NF | 477.3 |
| Polyethelene glycol 3350 NF | 23.9 |
| Propylene glycol USP | 5.5 |
| Polysorbate 80 NF | 0.6 |
| Carbopol 934-P | 1.0 |
| Fumed silica | 2.7 |
| Acetaminophen powder | 500.0 |

The fumed silica used was Cab-o-Sil M-5, which is a tradename of the Cabot Corporation, Tuscola, Illinois 61953. The acetaminophen powder used all passed through an 80 mesh sieve screen. The fill material was thoroughly mixed and encapsulated in conventional soft elastic gelatin shells having a volume of 13.4 minims to form soft elastic gelatin capsules. The fill material was nondilatant and compatibe with conventional soft elastic gelatin capsule manufacturing processes. Additionally, the gelled fill material was temperature stable and did not remove excessive amounts of water or plasticizers from the soft elastic gelatin shell.

Example 2

A comparative study of the capsules prepared to Example 1 was conducted by the Department of

18    0152292

Pharmaceutical Sciences, Medical University of South Carolina with respect to two leading powder filled, hard shell capsule acetaminophen formulations: Extra Strength Tylenol manufactured by McNeil Pharmaceutical and Panadol manufactured by Sterling-Winthrop Laboratories. The study design employed a single 500 mg. dose of each formulation in a random, 3-way cross-over study in which 12 healthy volunteers were employed, four subjects in each group. Data comparisons were made among the three tested products and a seven-day washout period separated each treatment.

A single 500 milligram oral dose of acetaminophen was administered following an overnight fast. The fasting continued for the four hour period following the acetaminophen administration. The subjects were instructed to avoid the use of all medications for 72 hours prior to the beginning of the study and for the duration of the study. They were also required to abstain from alcohol consumption for the 48 hours prior to and during the study. The subjects were instructed to fast from midnight on each of the three nights prior to administration of their single dose. Each subject then received his dose at a prescheduled time and plasma samples were obtained prior to dosing and at 15, 30, 60, 90, 120, 180, 240, 360, 480, 600, and 720 minutes after oral administration.

Below, as Table I, is a summary of the time required to reach maximum plasma acetaminophen concentration ($T_{max}$) and the maximum plasma acetaminophen concentration ($C_{max}$) for each of the products tested. Though the maximum plasma concentrations were not significantly different for the three products, the inventive soft elastic gelatin capsules had a significantly shorter time required to reach maximum plasma concentration. The $T_{max}$ value of the inventive capsule was 36.25 minutes while the two hard shell capsule formulations both had $R_{max}$ values of 63.75 minutes. Since the $R_{max}$ value is directly related to the onset of the therapeutic effects, the novel soft elastic gelatin capsules have a more rapid onset time than conventional hard shell capsule products. This rapid onset is particularly beneficial to patients seeking the quickest possible relief from pain or fever.

## Table 1

Summary of Pharmacokinetic Parameters for Each

Product Following Administration of a Single

Oral Dose of 500 mg of Acetaminophen

| Product | $C_{max}$ (ug/ml) | $T_{max}$ (min) |
|---|---|---|
| 1. Inventive | | |
| Example 1 | 8.78 | 36.25 |
| (S.D.) | (4.76) | (18.6) |
| Range | 4.43-21.50 | 15 - 60 |
| | | |
| 2. Extra-Strength | | |
| Tylenol | 7.91 | 63.75 |
| (S.D.) | (3.20) | (46.6) |
| Range | 3.72-13.45 | 15 - 180 |
| | | |
| 3. Panadol | 8.69 | 63.75 |
| (S.D.) | (6.40) | (36.8) |
| Range | 3.24-27.30 | 15 - 20 |

M&C FOLIO: 79949593                    WANGDOC: 0224i

CLAIMS:-(for contracting states BE, FR, DE, GB, IT, NL, SE AND CH)

1. A gelatin capsule for oral administration comprises a gelatin shell substantially encapsulating a physiologically compatible fill material, the fill material comprising an effective dosage amount of acetaminophen, a vehicle for the acetaminophen, a surfactant and a modifier for the vehicle, characterised in that:-

(a) the vehicle comprises a low molecular weight polyethylene glycol having an average molecular weight of from about 400 to 1000 and a higher molecular weight polyethylene glycol having an average molecular weight of from about 1000 to about 10000, the acetaminophen being substantially evenly distributed through the vehicle;

(b) the surfactant is of a type that is physiologically solubile and is in solution in the vehicle; and

(c) the viscosity modifier is present in an amount equal to or greater than the amount necessary to gel the vehicle.

2.   A capsule as claimed in claim 1 characterised in that the shell is a soft elastic gelatin shell and the fill material is non-dilatant.

3.   A capsule as claimed in claim 2 characterised in that the weight ratio of the low molecular weight polyethylene glycol to the higher molecular weight polyethylene glycol is from about 1:1 to about 40:1.

4.   A capsule as claimed in claim 3 characterised in that the said weight ratio is from about 15:1 to about 25:1, preferably about 20:1.

5.   A capsule as claimed in any one of claims 2-4 characterised in that the film material additionally comprises propylene glycol and/or glycerine in an amount equal to or less than about 10% by weight of the fill material.

6.   A capsule as claimed in claim 1 characterised in that the shell is a hard gelatin shell.

7.   A capsule as claimed in claim 6 characterised in that the weight ratio of the low molecular weight polyethylene glycol to the higher molecular weight polyethylene glycol is from about 0:1 to about 20:1.

23             **0152292**

8. A capsule as claimed in claim 7 characterised in that the said weight ratio is from 0:1 to about 15:1 preferably about 10:1.

9. A capsule as claimed in any one of the preceding claims characterized in that the low molecular weight polyethylene glycol has an average molecular weight of from about 400 to about 600.

10. A capsule as claimed in claim 9 characterized in that the low molecular polyethylene glycol has an average molecular weight of about 400.

11. A capsule as claimed in any one of the preceding claims characterised in that the higher molecular weight polyethylene glycol has an average molecular weight of from about 1450 to about 8000.

12. A capsule as claimed in claim 11 characterised in that the higher molecular weight polyethylene glycol has an average molecular weight of about 3350.

13. A capsule as claimed in any one of the preceding claims characterised in that the surfactant is present in an amount of from about 0.01 to about 1% by weight of the total weight of the fill material.

14. A capsule as claimed in any one of the preceding claims characterised in that the surfactant is polysorbate 80.

15. A capsule as claimed in claim 14 characterised in that the polysorbate 80 is present in an amount of about 0.5% by weight of the total weight of the fill material.

16. A capsule as claimed in any one of the preceding claims characterised in that the viscosity modifier is an acrylic acid resin having a high carboxylic content and an average molecular weight of approximately 3,000,000.

17. A capsule as claimed in claim 16 characterised in that the viscosity modifier is present in an amount of upto 1% by weight based on the total weight of the fill material.

18. A capsule as claimed in claim 16 characterised in that the viscosity modifier additionally comprises fumed silica.

19. A capsule as claimed in claim 18 characterised in that the acrylic resin is at present in an amount of about 0.1% by weight and the fumed silica in an amount of about 0.3% by weight, both based on the total weight of the film material.

20. A capsule as claimed in any one of the preceding claims characterised in that the viscosity modifier is fumed silica.

21. A capsule as claimed in claim 20 characterised in that the fumed silica is present in an amount of upto about 3% by weight based on the total weight of the fill material.

22. A capsule as claimed in any one of the preceding claims in which the acetaminophen has a particle size equal to or less than about US standard 80 mesh.

23. A capsule as claimed in any one of the preceding claims characterised in that acetaminophen is present in an amount of about 50% by weight based on the total weight of the film material.

24. A capsule as claimed in any one of the preceding claims containing approximately 500 mg of acetaminophen.

M&C FOLIO: 799P49593                    WANGDOC:0219i

CLAIMS:-(for contracting state AT)

1. A method for the preparation of a gelatin capsule for oral administration which comprises encapsulating in a gelatin shell a physiologically compatible fill material, the fill material comprising an effective dosage amount of acetaminophen, a vehicle for the acetaminophen, a surfactant and a modifier for the vehicle, characterised in that:-

(a)    the vehicle comprises a low molecular weight polyethylene glycol having an average molecular weight of from about 400 to 1000 and a higher molecular weight polyethylene glycol having an average molecular weight of from about 1000 to about 10000, the acetaminophen being substantially evenly distributed through the vehicle;

(b)    the surfactant is of a type that is physiologically soluble and is in solution in the vehicle; and

(c)    the viscosity modifier is present in an amount equal to or greater than the amount necessary to gel the vehicle.

2. A method as claimed in claim 1 characterised in that the shell is a soft elastic gelatin shell and the fill material is non-dilatant.

3. A method as claimed in claim 2 characterised in that the weight ratio of the low molecular weight polyethylene glycol to the higher molecular weight polyethylene glycol is from about 1:1 to about 40:1.

4. A method as claimed in claim 3 characterised in that the said weight ratio is from about 15:1 to about 25:1, preferably about 20:1.

5. A method as claimed in any one of claims 2-4 characterised in that the film material additionally comprises propylene glycol and/or glycerine in an amount equal to or less than about 10% by weight of the fill material.

6. A method as claimed in claim 1 characterised in that the shell is a hard gelatin shell.

7. A method as claimed in claim 6 characterised in that the weight ratio of the low molecular weight polyethylene glycol to the higher molecular weight polyethylene glycol is from about 0:1 to about 20:1.

8. A method as claimed in claim 7 characterised in that the said weight ratio is from 0:1 to about 15:1 preferably about 10:1.

9. A method as claimed in any one of the preceding claims in which the low molecular weight polyethylene glycol has an average molecular weight of from about 400 to about 600.

10. A method as claimed in claim 9 in which the low molecular polyethylene glycol has an average molecular weight of about 400.

11. A method as claimed in any one of the preceding claims characterised in that the higher molecular weight polyethylene glycol has an average molecular weight of from about 1450 to about 8000.

12. A method as claimed in claim 11 characterised in that the higher molecular weight polyethylene glycol has an average molecular weight of about 3350.

13. A method as claimed in any one of the preceding claims characterised in that the surfactant is present in an amount of from about 0.01 to about 1% by weight of the total weight of the fill material.

14. A method as claimed in any one of the preceding claims characterised in that the surfactant is polysorbate 80.

15. A method as claimed in claim 14 characterised in that the polysorbate 80 is present in an amount of about 0.5% by weight of the total weight of the fill material.

16. A method as claimed in any one of the preceding claims characterised in that the viscosity modifier is an acrylic acid resin having a high carboxylic content and an average molecular weight of approximately 3,000,000.

17. A method as claimed in claim 16 characterised in that the viscosity modifier is present in an amount of upto 1% by weight based on the total weight of the fill material.

18. A method as claimed in claim 16 characterised in that the viscosity modifier additionally comprises fumed silica.

19. A method as claimed in claim 18 characterised in that the acrylic resin is at present in an amount of about 0.1% by weight and the fumed silica in an amount of about 0.3% by weight, both based on the total weight of the fill material.

20. A method as claimed in any one of the preceding claims characterised in that the viscosity modifier is fumed silica.

21. A method as claimed in claim 20 characterised in that the fumed silica is present in an amount of upto about 3% by weight based on the total weight of the fill material.

22. A method as claimed in any one of the preceding claims in which the acetaminophen has a particle size equal to or less than about US standard 80 mesh.

23. A method as claimed in any one of the preceding claims characterised in that acetaminophen is present in an amount of about 50% by weight based on the total weight of the fill material.

24. A method as claimed in any one of the preceding claims containing approximately 500 mg of acetaminophen.